# EUROPÄISCHE PATENTSCHRIFT

(11) **EP 1 424 068 B1**
(45) Veröffentlichungstag und Bekanntmachung des Hinweises auf die Patenterteilung: **02.08.2006**
(21) Anmeldenummer: 02026447.9
(22) Anmeldetag: 27.11.2002
(51) Int. Cl.: A61K 9/16, A61K 9/50

(54) **Verfahren zur Herstellung von Pellets, enthaltend ein tri- oder tetracyclisches Antidepressivum sowie diese Pellets enthaltende pharmazeutische Zubereitungen**
Process for the preparation of pellets comprising a tricyclic or tetracyclic antidepressant and pharmaceutical compositions comprising these pellets
Procedé pour la préparation de pellets comprenant un antidepresseur tricyclique ou tetracyclique et compositions pharmaceutiques comprenant ces pellets

(43) Veröffentlichungstag der Anmeldung: 02.06.2004
(73) Patentinhaber: ORAMON-Arzneimittel GmbH & Co. KG, 88471 Laupheim (DE)
(72) Erfinder: Heinz, Carmen, 88471 Laupheim (DE); Neuer, Klaus, 88471 Laupheim (DE); Walch, Hatto, Dr., 88471 Laupheim (DE)
(74) Vertreter: Schwabe - Sandmair - Marx

(56) Entgegenhaltungen:
- WO-A-98/04249
- FR-A- 2 313 915
- US-A- 4 874 613

## Beschreibung

Die vorliegende Erfindung betrifft ein Verfahren zur Herstellung von Pellets, welche ein tri- oder tetracyclisches Antidepressivum, vorzugsweise Amitriptylin, enthalten sowie pharmazeutische Zubereitungen in Form von nach diesem Verfahren hergestellten Pellets.

### Stand der Technik

Tri- und tetracyclische Antidepressiva werden heutzutage verbreitet zur Therapie oder Linderung der unter der Bezeichnung "depressives Syndrom" angesprochenen psychischen, somatischen oder psychosozialen Symptome verwendet. Psychische Symptome des depressiven Syndroms sind unter anderem die niedergeschlagene Verstimmung, die Freudlosigkeit, Interesselosigkeit, Energielosigkeit wie auch Angstzustände, Zwänge, Konzentrationsstörungen und Ähnliches. Somatisch ist das depressive Syndrom häufig gekennzeichnet durch Schlaf- und Appetitstörungen, Gewichtsverlust, gastrointestinale Beschwerden, Kopfschmerz, Kreislaufstörungen aber auch diffuse Beschwerden im Bereich von Muskulatur und Skelettsystem, Sekretionsstörungen oder Beeinträchtigung von Stimme, Psychomotorik und ähnlichem. Das depressive Symptom geht zumeist auch mit psychosozialen Störungen einher, wie dem Rückgang zwischenmenschlicher Kontakte, der Isolationsneigung und allgemeinen Problemen im Umgang mit Dritten.

Die oben genannten, unter der Bezeichnung depressives Syndrom angesprochenen Symptome können psychogenen, endogenen, somatogenen oder anaklitischen Ursprungs sein.

Therapiert werden diese Symptome heutzutage unter anderem durch die Verabreichung von Antidepressiva. Diese können antriebssteigernd und stimmungsaufhellend oder anxiolytisch und antriebsdämpfend wirken. Verwendet werden vor allem tricyclische Antidepressiva wie das Amitriptylin, Amitriptylinoxid, Clomipramin, das Desipramin, das Dibenzepin, das Doxepin, das Ipramin, das Lofepramin, das Melitracen, das Nortriptylin, das Noxiptilin, das Protriptylin, das Trimipramin und andere und tetracyclische Antidepressiva wie beispielsweise das Maprotilin, Mianserin, Nomifensin und andere.

Obwohl der exakte Wirkmechanismus der genannten Antidepressiva noch nicht geklärt ist, wird angenommen, dass diese in den Stoffwechsel der Monoamine eingreifen. Konkret liegen Hinweise darauf vor, dass die Substanzen die Wiederaufnahme von Noradrenalin und/oder Serotonin aus dem synaptischen Spalt im Axoplasma hemmen. Eine Hemmung der Noradreanlin-Wiederaufnahme wird mit einer Antriebssteigerung, eine Hemmung der Serotonin-Wiederaufnahme mit einer Stimmungsaufhellung in Zusammenhang gebracht. Bei den einzelnen Substanzen ist die Stärke der Rücktransporthemmung bzw. der Wiederaufnahmehemmung unterschiedlich stark ausgeprägt, wobei die Befunde zur Hemmungsneigung für Noradrenalin bzw. Serotonin mit dem gefundenen Wirkprofil der jeweiligen Substanz übereinstimmen.

Die Antidepressiva werden üblicherweise oral in Dosen zwischen 10 und 400 mg pro Tag verabreicht, wobei die Dosierung selbstverständlich dem Einzelfall angepasst ist.

Bei den tri- und tetracyclischen Antidepressiva handelt es sich um substituierte Diphenylamin- und Diphenylmethanderivate, in denen die Phenylsubstituenten zweifach verbrückt sind, so dass die Gesamtringsysteme stark gewinkelte Strukturen aufweisen. Beispielsweise handelt es sich bei einem der bevorzugt verabreichten Antidepressiva, dem Amitriptylin, um ein Dibenzocycloheptadienderivat. Auf Grund der stark gewinkelten Strukturen neigen alle tri- und tetracyclischen Antidepressiva zur Zersetzung, insbesondere bei Einwirkung von Feuchtigkeit und/oder Wärme. Diese Zersetzungsprodukte sind in fertigen Arzneiformulierungen unerwünscht. So schreibt beispielsweise die europäische Pharmakopoe maximale Gehalte an Zersetzungsprodukten vor. Es ist bekannt, dass diese von den am Markt befindlichen Formulierungen meist ausgeprägt überschritten werden.

Aufgabe der vorliegenden Erfindung ist es daher, eine stabile orale, vorzugsweise retardierte Arzneiform von tri- und tetracyclischen Antidepressiva zu entwickeln, wie auch ein Verfahren zu deren Herstellung.

### Kurze Beschreibung der Erfindung

Die erfindungsgemäße Aufgabe wird gelöst sowie weitere Nachteile des Standes der Technik überwunden durch ein Verfahren zur Herstellung von Pellets, enthaltend ein tri- oder tetracyclisches Antidepressivum, umfassend die Schritte:
(a) Vorlegen von Neutralpellets;
(b) Einstreuen des tri- oder tetracyclische Antidepressivums unter gleichzeitigen Aufsprühen einer wässrigen, nicht alkoholischen Schellacklösung ohne Zuluft zum Erhalt einer Wirkstoffschicht und gegebenenfalls Trocknen der Wirkstoffschicht, sowie
(c) wahlweise Aufbringen einer oder mehrerer weiterer Schichten, wobei die erste dieser Schichten (= Isolierschicht), die in Kontakt mit der Wirkstoffschicht steht, durch Aufsprühen einer wässrigen, nicht alkoholischen Schellacklösung erhalten wird.

Weiterer Gegenstand der Erfindung ist eine pharmazeutische Zubereitung in Form von Pellets, enthaltend in dieser Reihenfolge von innen nach außen (i) einen neutralen, festen Kern, (ii) eine Wirkstoffschicht, umfassend ein tri- oder tetracyclisches Antidepressivum, einen wasserlöslichen Schellack sowie ggf. einen Zucker und/oder ein Antioxidans, und (iii) wahlweise eine oder mehrere weitere Schichten, wobei die erste dieser weiteren Schichten, die in Kontakt mit der Wirkstoffschicht steht, einen wasserlöslichen Schellack enthält.

### Genaue Beschreibung der Erfindung

In einem ersten Aspekt derselben betrifft die vorliegende Erfindung somit ein Verfahren zur Herstellung von Pellets, enthaltend ein tri- oder tetracyclisches Antidepressivum, umfassend die Schritte:
(a) Vorlegen von Neutralpellets;
(b) Einstreuen des tri- oder tetracyclische Antidepressivums unter gleichzeitigem Aufsprühen einer wässrigen, nicht alkoholischen Schellacklösung ohne Zuluft zum Erhalt einer Wirkstoffschicht und gegebenenfalls Trocknen der Wirkstoffschicht, sowie
(c) wahlweise Aufbringen einer oder mehrerer weiterer Schichten, wobei die erste dieser Schichten (= Isolierschicht), die in Kontakt mit der Wirkstoffschicht steht, durch Aufsprühen einer wässrigen, nicht alkoholischen Schellacklösung erhalten wird.

Das erfindungsgemäße Verfahren zur Herstellung von ein tri- oder tetracyclisches Antidepressivum enthaltenden Pellets gestattet überraschend, das tri- oder tetracyclische Antidepressivum dauerhaft gegenüber Zersetzung zu stabilisieren, so dass die Reinheitskriterien der europäischen Pharmakopoe durch das Präparat eingehalten werden können. Konkret weisen die nach dem erfindungsgemäßen Verfahren hergestellten pharmazeutischen Zubereitungen in Form von Pellets, enthaltend ein tri- oder tetracyclisches Antidepressivum, die Gegenstand dieser Erfindung sind, beispielsweise für Amitriptylin als Antidepressivum weniger als 0,2 % der einzelnen Nebenprodukte und weniger als 0,05 % Dibenzosuberon als Hauptnebenprodukt auf, wobei die Summe aller Nebenprodukte kleiner als 0,5 % ist. Dies gilt auch für Tests während der Haltbarkeitsprüfung bei verschiedenen Temperaturen, konkret bei 25 °C/60 % relative Feuchte und 40 °C/75 % relative Feuchte.

Die vorgenannten Grenzwerte wurden bei der Herstellung von Pellets auf konventionelle Weise bereits nach kurzer Lagerdauer deutlich überschritten. Dies gilt auch für Pellets, die nach dem klassischen Aufbauverfahren hergestellt werden, das heißt Vorlage von Neutralpellets und kontinuierlichem Einsprühen einer ethanolischen Schellacklösung, verbunden mit Einstreuung des Amitriptylins in Pulverform und Zwischentrocknungen.

Erfindungsgemäß wird grundsätzlich ebenfalls nach dem klassischen Aufbauverfahren ausgehend von Neutralpellets gearbeitet. Wesentlich ist erfindungsgemäß jedoch, dass das tri- oder tetracyclische Antidepressivum eingestreut und mit Hilfe einer als Bindemittel wirkenden wässrigen, nicht alkoholischen Schellacklösung, die gleichzeitig aufgesprüht wird, auf den Neutralpellets fixiert wird und während des Aufsprühens ohne Zuluft gearbeitet wird. Klassisch werden als Bindemittel entweder Polyvidon, Polyacrylate oder Ethylcellulose, wie auch ethanolische Schellacklösungen verwendet. Gleichzeitig arbeitet man üblicherweise, zwecks schnellerer Trocknung, unter Zuluft, das heißt mit Sauerstoffzufuhr, beim Aufsprühen des Wirkstoffs. In allen diesen Fällen werden jedoch Pellets erhalten, bei denen die Grenzwerte für die Reinheitsanforderungen der europäischen Pharmakopoe deutlich überschritten werden.

Die erfindungsgemäße Verwendung einer wässrigen, nicht alkoholischen Schellacklösung als Bindemittel und vor allem das Arbeiten ohne Zuluft während des Einsprühens gestatten es nun überraschenderweise, das tri- oder tetracyclische Antidepressivum zu stabilisieren und dementsprechend die Reinheitskriterien gemäß europäischer Pharmakopoe einzuhalten.

Im Schritt (a) des erfindungsgemäßen Verfahrens werden im Kessel einer üblichen Apparatur zum Aufsprühen von Wirkstoffen Neutralpellets als Pelletkern vorgelegt. Derartige Neutralpellets sind bekannt und im Handel erhältlich. Sie enthalten bekannte Arzneimittelhilfsstoffe wie Siliziumdioxid, Carbonate, Cellulosen, Polyvidon, Mg-Stearat, Stärke, Zucker (Saccharose) usw., bevorzugt Saccharose/Maisstärke und/oder mikrokristalline Cellulose. Die Neutralpellets haben üblicherweise Größen im Bereich von 0,5 bis 5 mm, vorzugsweise 0,5 bis 1,5 mm und sind rund, oval oder ähnlich geformt.

Im Schritt (b) des erfindungsgemäßen Verfahrens wird auf diese Neutralpellets eine wässrige, nicht alkoholische Schellacklösung aufgesprüht, die das tri- oder tetracyclische Antidepressivum bindet. Das Antidepressivum wird während des Aufsprühens der wässrigen, nicht alkoholische Schellacklösung zur Bildung einer Wirkstoffschicht separat eingestreut. Das Einstreuen während des gesamten Aufsprühens ist erfindungsgemäß bevorzugt.

Erfindungswesentlich ist, dass das tri- oder tetracyclische Antidepressivum zusammen mit einer wässrigen, nicht alkoholischen Schellacklösung aufgetragen wird. Diese wässrige Schellaklösung kann eine salzhaltige, gepufferte Lösung darstellen. Wesentlich ist, dass es sich bei der Hauptmenge des Lösungsmittels um Wasser handelt. Am meisten bevorzugt ist die Verwendung von gereinigtem Wasser als einzigem Lösungsmittel. Die wässrige, nicht-alkoholische Schellacklösung kann andere übliche Bestandteile wie Weichmacher (beispielsweise Glycerol, Propandiol), Talkum, Zucker (z.B. Saccharose), Antioxidantien (z.B. Ascorbinsäure und deren Salze, Tocopherole), oder andere Bindemittel wie Polyvidon, Polyacrylate, Cellulosen usw. enthalten. Letztere können den Schellack auch ganz ersetzten. Bevorzugt sind keine anderen Bindemittel als Schellack enthalten.

Bei dem erfindungsgemäß verwendeten Schellack handelt es sich um einen wasserlöslichen Schellack, bevorzugt einen bei neutralem pH wasserlöslichen Schellack. Derartige Schellacke sind bekannt. Beispielsweise können die im Handel unter den Bezeichnungen Schellak 55B63 Hydram von der Firma Stroever erhältlichen Schellacke verwendet werden.

Die wässrige, nicht alkoholische Schellacklösung wird erfindungsgemäß auf die vorgelegten Neutralpellets aufgesprüht. Dies kann grundsätzlich mit Hilfe jeder geeigneten Apparatur erfolgen. Wesentlich ist, dass das Aufsprühen der wässrigen, nicht alkoholischen Schellacklösung zum Erhalt einer Wirkstoffschicht ohne Zuluft erfolgt. Dies bedeutet, dass während des Aufsprühens kein sauerstoffhaltiges Gas in die Apparatur eingeführt wird. Dadurch wird ein Kontakt des tri- oder tetracyclischen Antidepressivums mit Sauerstoff während der Herstellung der erfindungsgemäßen Pellets vermieden. Ohne hierdurch gebunden zu sein, scheint dieses für eine bessere Stabilisierung des Antidepressivums wesentlich zu sein. Parallel zum Aufsprühen dieser Lösung wird das Antidepressivum eingestreut. Dies kann über den gesamten Aufsprühvorgang erfolgen oder in dessen Verlauf zugestreut und/oder das Einstreuen vor Ende des Aufsprühens beendet werden.

Beispielsweise können die Neutralpellets vorgelegt werden in einem konventionellen schräg stehenden Dragierkessel aber auch einem horizontal stehenden Kessel wie einem Pellegrini-Kessel oder einem Accela-Coater.

Es wird ausschließlich die wässrige, nicht alkoholische Schellacklösung aufgesprüht. Das tri- oder tetracyclische Antidepressivum wird gleichzeitig eingestreut. Das Aufsprühen der wässrigen, nicht alkoholischen Schellacklösung und das Einstreuen des tri- oder tetracyclische Antidepressivums, kann mehrfach bis zum Erhalt des gewünschten Gehalts an Antidepressivum durchgeführt werden.

Nach Erhalt der Wirkstoffschicht im Schritt (b) des erfindungsgemäßen Verfahrens können wahlweise eine oder mehrere weitere Schichten vorzugsweise von Schellak in Form einer wässrigen Lösung aufgebracht werden. Dies erfolgt, um den Wirkstoff zu versiegeln und/oder das Freisetzungsprofil einzustellen. Die erste dieser Schichten, die in Kontakt mit der Wirkstoffschicht steht, wird erfindungsgemäß ebenfalls durch Aufsprühen einer wässrigen, nicht alkoholischen Schellacklösung erhalten, die jedoch keinen Wirkstoff enthält. Hierdurch wird eine Isolierschicht erhalten. Bevorzugt wird nach dem erfindungsgemäßen Verfahren im Schritt (c) wenigstens eine weitere Schicht als Isolierschicht durch Aufsprühen einer wässrigen, nicht alkoholischen Schellacklösung ohne Wirkstoff aufgebracht. Noch stärker bevorzugt werden weitere Schichten aufgesprüht, bei denen es nicht zwingend um Schellackschichten handeln muss. Bevorzugt wird durch Aufsprühen weiterer Schichten eine retardierte Freisetzungscharakteristik eingestellt. Diese weitere oder weiteren Schicht(en) können übliche Zusätze wie Weichmacher, Farbstoffe, Talkum, Zucker, Geschmacksstoffe, Antioxidantien und deren Mischungen enthalten.

Das Aufsprühen der wässrigen, nicht alkoholischen Schellacklösung erfolgt bevorzugt mit einer Sprührate von 2,5 bis 20, vorzugsweise 5 bis 10 g/Minute auf 1 bis 10 kg Neutralpellets, wobei das Antidepressivum gleichzeitg in einer Menge von 1 bis 20, vorzugsweise 5 bis 15 g/Minute eingestreut wird. Stärker bevorzugt ist ein Verfahren, worin zunächst eine wässrige, nicht alkoholische Schellacklösung mit einer Sprührate von 2,5 bis 20, vorzugsweise 5 bis 10 g/Minute auf 1 bis 10 kg Neutralpellets aufgetragen wird, nach 0,2 bis 3, vorzugsweise 0,5 bis 1,5 Minuten Sprühzeit das Antidepressivum in einer Menge von 1 bis 20, vorzugsweise 5 bis 15 g/Minute eingestreut wird. Am meisten bevorzugt wird anschließend auf die erhaltenen Pellets eine wässrige, nicht alkoholische Schellacklösung mit einer Sprührate von 1 bis 20, vorzugsweise 3 bis 10 g/Minute für 0,2 bis 3, vorzugsweise 0,5 bis 1,5 Minuten aufgebracht und danach, falls gewünscht, zum Erhalten der Isolierschicht getrocknet.

Die erfindungsgemäß verwendeten tri- oder tetracyclischen Antidepressiva sind die üblicherweise verwendeten, dem Fachmann bekannten Substanzen. Selbstverständlich können auch Mischungen dieser Wirkstoffe verwendet werden. Das tricyclische Antidepressivum ist vorzugsweise ausgewählt aus der Gruppe, bestehend aus Iminodibenzylderivaten, Iminostilbenderivaten, Dibenzocycloheptadienderivaten, Dibenzocycloheptatrienderivaten, Dibenzodiazepinderivaten, Dibenzoxepinderivaten Dihydroanthracenderivaten und deren pharmazeutisch annehmbaren Säureadditionssalzen.

Selbstverständlich können auch substituierte Derivate wie halogenierte Derivate, alkylierte Derivate, mit Nitrogruppen versehene Derivate usw. der genannten Substanzen verwendet werden. Am meisten bevorzugt ist das tricyclische Antidepressivum ausgewählt aus der Gruppe, bestehend aus Imipramin, Desipramin, Trimipramin, Lofepramin, Clomipramin, Opipramol, Amitriptylin, Amitriptylinoxid, Nortriptylin, Noxiptilin, Protriptylin, Dibenzepin, Doxepin, Melitracen, deren Additionssalzen mit einer Halogenwasserstoffsäure, insbesondere HCI, oder Äpfelsäure und Mischungen derselben.

Am meisten bevorzugt ist das Antidepressivum Amitriptylin, Amitriptylinoxid, Amitriptylin-HCl oder Amitriptylinoxid-HCl.

Das tetracyclische Antidepressivum ist vorzugsweise ausgewählt unter Mianserin und Maprotin.

Gemäß dem zweiten Aspekt betrifft die vorliegende Erfindung pharmazeutische Zubereitungen in Form von Pellets, die nach dem erfindungsgemäßen Verfahren erhalten werden. Bevorzugt betrifft die Erfindung somit eine pharmazeutische Zubereitung in Form von Pellets, enthaltend in dieser Reihenfolge von innen nach außen (i) einen neutralen, festen Kern, (ii) ggf. eine erste Unterschicht aus einem wasserlöslichen Schellack, (iii) eine Wirkstoffschicht, umfassend ein tri- oder tetracyclisches Antidepressivum, einen wasserlöslichen Schellack und gegebenenfalls einen Zucker und/oder ein Antioxidans, und (iv) wahlweise eine oder mehrere weitere Schichten, wobei die erste dieser weiteren Schichten, die in Kontakt mit der Wirkstoffschicht steht, einen wasserlöslichen Schellack enthält.

Bevorzugt ist eine pharmazeutische Zubereitung, worin jedes Pellet enthält: (i) einen Kern, (ii) gegebenenfalls eine erste Unterschicht, (iii) eine Wirkstoffschicht, enthaltend Amitriptylin, Amitriptylinoxid, Amitriptylin-HCl oder Amitriptylinoxid-HCl als Wirkstoff, wasserlöslichen Schellack, Zucker und gegebenenfalls ein Oxidans, und (iv) mindestens eine weitere Schicht (= Isolierschicht). Am meisten bevorzugt liegt die pharmazeutische Zubereitung als Retardformulierung vor.

Die pharmazeutische Zubereitung kann die Pellets als solche enthalten. Die Pellets können zusätzlich in Kapseln oder Sachetts enthalten sein, um die Dosierung zu erleichtern. Die Pellets können gleichermaßen mittels weiterer Hilfsstoffe zu Unit-Size-Tabletten verpresst sein, sofern hierdurch die Beschichtung der Pellets nicht wesentlich beschädigt wird.

Am meisten bevorzugt ist eine pharmazeutische Zubereitung, worin jedes Pellet auf 406 bis 500 mg/g Neutralpelletkern enthält: 20 bis 400 mg/g Antidepressivum, 5 bis 35 mg/g wasserlöslichen Schellack, gegebenenfalls 50 bis 200 mg/g Talkum und gegebenenfalls 0,5 bis 2,5 mg/g Zucker.

Wie oben beschrieben, können alle Schellackschichten weitere Bestandteile, wie oben definiert, enthalten. Bevorzugt sind Wirkstoffschichten, die zusätzlich Talkum, Zucker und/oder Antioxidantien enthalten. Ebenso bevorzugt sind Pellets, die zusätzlich weitere äußere Schichten auf der Isolierschicht, wie oben beschrieben, aufweisen.

Die folgenden Beispiele sollen die vorliegende Erfindung veranschaulichen, diese jedoch nicht beschränken:

### BEISPIEL

### Beispiel 1:

In diesem Beispiel wurden 327 g Schellack, wasserlöslich SSB 63 Hydram, in 1.600 g gereinigtem Wasser gelöst. Im Kessel versehen mit einer Sprüheinrichtung werden 5.090 g Neutralpellets mit einem Durchmesser von 0,5 - 0,6 mm vorgelegt. Die Schellacklösung wird auf die Neutralpellets mit einer Sprührate von 5 bis 10 g/Minute aufgesprüht. Nach einer Minute Sprühzeit wurde kontinuierlich eine Menge von 210 - 280 g einer Mischung am Amitriptyhin und Talkum im Verhältnis 2 : 1 eingestreut. Das Aufsprühen der Schellack-Lösung erfolgt für 15 - 20 Minuten. Danach erfolgt eine Zwischentrocknung für 10 - 20 Minuten ohne Zufuhr von Trockenluft. Während des gesamten Prozesses, der in einem herkömmlichen Dragierkessel durchgeführt wurde, wurde keine Zuluft eingeblasen. Die Wirkstoffpellets wurden nach vollständigem Wirkstoffauftrag und Versiegeln mit wässriger Schellack-Lösung entnommen und 24 Stunden zwischengetrocknet.

Anschließend wurde eine Retardschicht für Wirkstoffpellets in einer Wirbelschichtapparatur aufgetragen. Hierzu wurden wiederum 76 g Schellack, wasserlöslich SSB 63 Hydram, in 637 g gereinigtem Wasser gelöst, dann 3,8 g Glycerol als Weichmacher und anschließend 7,6 g Talkum suspendiert. 1000 g der Wirkstoffpellets wurden vorgelegt und die Schellacksuspension kontinuierlich mit einer Sprührate von 3 bis 10 g/Minute bei einer Zulufttemperatur von 40 bis 45 °C, einer Ablufttemperatur von 30 bis 32 °C für 190 Minuten aufgesprüht. Die Pellets werden 24 Stunden nachgetrocknet.

Die fertigen Pellets wurden für eine Zeitspanne von 6 Monaten gelagert (25 °C/60 % relative Feuchte bzw. 40 °C/75 % relative Feuchte) und anschließend auf Zersetzung des Produkts getestet. Sowohl in Pellets ohne Überzug als auch in Pellets mit Überzug konnten nach Lagerung keine Nebenprodukte einzeln und in der Summe nachgewiesen werden, die in der Menge über den zulässigen Grenzwerten lagen. Dies gilt auch für Dibenzosuberon. Konkret lagen die nach diesem Verfahren hergestellten Pellets bezüglich der Reinheit für die einzelnen Nebenprodukte bei 0,03 % (Grenzwert 0,2 %), in der Summe bei 0,05 bis 0,08 % (Grenzwert 0,5 %) und bezüglich Dibenzosuberon bei <0,05 % (Grenzwert <0,05 %).

Die fertigen Wirkstoffpellets wiesen die folgende Zusammensetzung auf:

| Formulierung Nr. 1 | |
|---|---|
| | mg/g Pellets |
| Amitriptylin | 290,423 |
| Neutralpellets* | 494,996 |
| Schellack wasserlöslich SSB 63 Hydram | 98,140 |
| Talkum | 152,182 |
| Glycerol | 3,485 |
| | 1000,000 |

- * bestehend aus: Saccharose 80 %
Maisstärke 20 %

### Beispiel 2

Nach dem Verfahren von Beispiel 1 wurden Pellets folgender Zusammensetzung hergestellt:

| Formulierung Nr. 2 | |
|---|---|
| Amitriptylin | 75,000 mg |
| Neutralpellets* | 117,500 mg |
| Talkum | 37,500 mg |
| Ascorbinsäure | 1,125 mg |
| Schellack | 7,110 mg |

| Formulierung Nr. 3 | | |
|---|---|---|
| | Amitriptylin | 75,00 mg |
| | Neutralpellets* | 117,50 mg |
| | Talkum | 37,50 mg |
| | Schellack | 7,11 mg |
| (Isolierschicht) | Schellack | 1,00 mg |
| | Talkum | 4,00 mg |
| (Retardlack) | Ethylcellulose | 24,00 mg |
| | Neutralöl | 5,10 mg |
| | Talkum | 37,50 mg |

| Formulierung Nr. 4 | | |
|---|---|---|
| | Amitriptylin | 75,00 mg |
| | Neutralpellets* | 117,50 mg |
| | Talkum | 37,50 mg |
| | Schellack | 7,11 mg |
| (Isolierschicht) | Schellack | 1,00 mg |
| | Talkum | 4,00 mg |
| (Retardlack) | Polyvinylacetat/Polyvinylpyrrolidon 90:10 | 24,00 mg |
| | Propandiol | 0,55 mg |
| | Talkum | 2,20 mg |
| | Talkum | 37,50 mg |

## Patentansprüche

1. Verfahren zur Herstellung von Pellets, enthaltend ein tri- oder tetracyclisches Antidepressivum, umfassend die Schritte:
(a) Vorlegen von Neutralpellets;
(b) Einstreuen des tri- oder tetracyclischen Antidepressivums und gleichzeitiges Aufsprühen einer wässrigen, nicht alkoholischen Schellacklösung ohne Zuluft zum Erhalt einer Wirkstoffschicht und gegebenenfalls Trocknen der Wirkstoffschicht, sowie
(c) wahlweise Aufbringen einer oder mehrerer weiterer Schichten, wobei die erste dieser Schichten (= Isolierschicht), die in Kontakt mit der Wirkstoffschicht steht, durch Aufsprühen einer wässrigen, nicht alkoholischen Schellacklösung erhalten wird.

2. Verfahren nach Anspruch 1, worin das Antidepressivum in Schritt (b) während des gesamten Aufsprühens der wässrigen, nicht alkoholischen Schellacklösung eingestreut wird.

3. Verfahren nach Anspruch 1, worin die wässrige, nicht alkoholische Schellacklösung in Schritt (b) auf die bewegten Neutralpellets im Dragierkessel aufgesprüht wird.

4. Verfahren nach einem der vorstehenden Ansprüche, worin Schritt (b) bis zum Erhalt des gewünschten Gehalts an Antidepressivum pro Pellet mehrfach durchgeführt wird.

5. Verfahren nach einem der vorstehenden Ansprüche, worin vor dem Aufsprühen der Wirkstoffschicht in Schritt (b) eine wässrige, nicht alkoholische Schellacklösung auf die Neutralpellets zur Bildung einer Unterschicht aufgebracht und ggf. getrocknet wird.

6. Verfahren nach einem der vorstehenden Ansprüche, worin in Schritt (c) eine weitere Schicht durch Aufsprühen einer wässrigen, nicht alkoholischen Schellacklösung ohne Wirkstoff aufgebracht wird.

7. Verfahren nach einem der Ansprüche 1 bis 5, worin in Schritt (c) durch Aufsprühen einer oder mehrerer weiterer Schichten eine retardierte Freisetzungscharakteristik eingestellt wird.

8. Verfahren nach einem der vorstehenden Ansprüche, worin das tricyclische Antidepressivum ausgewählt ist aus der Gruppe, bestehend aus Iminodibenzylderivaten, Iminostilbenderivaten, Dibenzocycloheptadienderivaten, Dibenzocycloheptatrienderivaten, Dibenzodiazepinderivaten, Dibenzoxepinderivaten Dihydroanthracenderivaten und deren pharmazeutisch annehmbaren Säureadditionssalzen.

9. Verfahren nach Anspruch 8, worin das Antidepressivum ausgewählt ist aus der Gruppe, bestehend aus Imipramin, Desipramin, Trimipramin, Lofepramin, Clomipramin, Opipramol, Amitriptylin, Amitriptylinoxid, Nortriptylin, Noxiptilin, Protriptylin, Dibenzepin, Doxepin, Melitracen, deren Additionssalzen mit einer Halogenwasserstoffsäure, insbesondere HCl, und Mischungen derselben.

10. Verfahren nach Anspruch 9, worin das Antidepressivum Amitriptylin, Amitriptylinoxid, Amitriptylin-HCl oder Amitriptylinoxid-HCl ist.

11. Verfahren nach einem der vorstehenden Ansprüche, worin die in Schritt (b) verwendet Schellacklösung einen wasserlöslichen Schellack enthält.

12. Verfahren nach einem der vorstehenden Ansprüche, worin die Schellacklösung zusätzlich einen Zucker, vorzugsweise Saccharose, Talkum und/oder ein Antioxidans enthält.

13. Verfahren nach einem der vorstehenden Ansprüche, worin zunächst eine wässrige, nicht alkoholische Schellacklösung mit einer Sprührate von 2,5 bis 20, vorzugsweise 5 bis 10 g/min auf 1000 - 104 g Neutralpellets aufgetragen wird, und nach 0,2 bis 3, vorzugsweise 0,5 bis 1,5 Minuten Sprühzeit das Antidepressivum in einer Menge von 1 bis 20, vorzugsweise 5 bis 15 g/min eingestreut wird.

14. Verfahren nach Anspruch 13, worin auf die erhaltenen Pellets anschließend eine wässrige, nicht alkoholische Schellacklösung mit einer Sprührate von 1 bis 20, vorzugsweise 3 bis 10 g/min für 0,2 bis 3, vorzugsweise 0,5 bis 1,5 Minuten aufgebracht und anschließend zum Erhalt einer Isolierschicht getrocknet wird.

15. Verfahren nach einem der vorstehenden Ansprüche, worin die weitere(n) Schellackschicht(en) übliche Zusätze wie Weichmacher, Farbstoffe, Zucker, Geschmacksstoffe, Antioxidantien etc. enthalten.

16. Verfahren nach einem der vorstehenden Ansprüche, worin der wässrige Schellack als Bindemittel ganz oder teilweise ersetzt wird durch Povidon, Polyacrylate, Cellulose, modifizierte Cellulose und deren Mischungen.

17. Pharmazeutische Zubereitung in Form von Pellets, enthaltend in dieser Reihenfolge von innen nach außen (i) einen neutralen, festen Kern, (ii) ggf. eine erste Unterschicht aus einem wasserlöslichen Schellack, (iii) eine Wirkstoffschicht, umfassend ein tri-oder tetracyclisches Antidepressivum, einen wasserlöslichen Schellack sowie ggf. einen Zucker, und (iv) wahlweise eine oder mehrere weitere Schichten, wobei die erste dieser weiteren Schichten, die in Kontakt mit der Wirkstoffschicht steht, einen wasserlöslichen Schellack enthält.

18. Pharmazeutische Zubereitung nach Anspruch 17, worin jedes Pellet enthält: (i) einen Kern, (ii) eine Wirkstoffschicht, enthaltend Amitriptylin, Amitriptylinoxid, Amitriptylin-HCl oder Amitriptylinoxid-HCl als Wirkstoff, wasserlöslichen Schellack und Zucker, und (iii) mindestens eine weitere Schicht.

19. Pharmazeutische Zubereitung nach Anspruch 17 oder 18 als Retardformulierung.

20. Pharmazeutische Zubereitung nach Anspruch 17, 18 oder 19, worin jedes Pellet auf 406 bis 500 mg/g Neutralpelletkem enthält: 20 bis 400 mg/g Antidepressivum, 5 bis 35 mg/g wasserlöslichen Schellack, gegebenenfalls 50 bis 200 mg/g Talkum und gegebenenfalls 0,5 bis 2,5 mg/g Zucker.

## Claims

1. Process for the preparation of pellets comprising a tri- or tetracyclic antidepressant which comprises the stages:
(a) introducing neutral pellets;
(b) disseminating the tri- or tetracyclic antidepressants and simultaneous spraying on an aqueous nonalcoholic shellac varnish without incoming air, in order to obtain an active agent layer, and, if appropriate, drying the active agent layer, and
(c) optionally applying one or more additional layers, the first of these layers (= isolating layer), which is in contact with the active agent layer, being obtained by spraying on an aqueous nonalcoholic shellac varnish.

2. Process according to Claim 1, in which the antidepressant in stage (b) is disseminated during the entire spraying on of the aqueous nonalcoholic shellac varnish.

3. Process according to Claim 1, in which the aqueous nonalcoholic shellac varnish in stage (b) is sprayed onto the agitated neutral pellets in the coating pan.

4. Process according to one of the preceding claims, in which stage (b) is carried out repeatedly until the desired content of antidepressant per pellet is obtained.

5. Process according to one of the preceding claims, in which, before spraying on the active agent layer in stage (b), an aqueous nonalcoholic shellac varnish is applied to the neutral pellets, in order to form a lower layer, and, if appropriate, is dried.

6. Process according to one of the preceding claims, in which, in stage (c), an additional layer is applied by spraying on an aqueous nonalcoholic shellac varnish without active agent.

7. Process according to one of Claims 1 to 5, in which, in stage (c), a delayed release property is introduced by spraying on one or more additional layers.

8. Process according to one of the preceding claims, in which the tricyclic antidepressant is chosen from the group consisting of iminodibenzyl derivatives, iminostilbene derivatives, dibenzocycloheptadiene derivatives, dibenzocycloheptatriene derivatives, dibenzodiazepine derivatives, dibenzoxepin derivatives, dihydroanthracene derivatives and the pharmaceutically acceptable acid addition salts thereof.

9. Process according to Claim 8, in which the antidepressant is chosen from the group consisting of imipramine, desipramine, trimipramine, lofepramine, clomipramine, opipramol, amitriptyline, amitriptylinoxide, nortriptyline, noxiptilin, protriptyline, dibenzepin, doxepin, melitracen, the addition salts thereof with a hydrohalic acid, in particular HCl, and mixtures of the same.

10. Process according to Claim 9, in which the antidepressant is amitriptyline, amitriptylinoxide, amitriptyline-HCl or amitriptylinoxide-HCl.

11. Process according to one of the preceding claims, in which the shellac varnish used in stage (b) comprises a water-soluble shellac.

12. Process according to one of the preceding claims, in which the shellac varnish additionally comprises a sugar, preferably sucrose, talc and/or an antioxidant.

13. Process according to one of the preceding claims, in which first an aqueous nonalcoholic shellac varnish is applied with a spraying rate of 2.5 to 20 g/min, preferably 5 to 10 g/min, to 1000 - 10⁴ g of neutral pellets and, after 0.2 to 3 minutes spraying time, preferably 0.5 to 1.5 minutes spraying time, the antidepressant is disseminated in an amount of 1 to 20 g/min, preferably 5 to 15 g/min.

14. Process according to Claim 13, in which an aqueous nonalcoholic shellac varnish is subsequently applied with a spraying rate of 1 to 20 g/min, preferably 3 to 10 g/min, for 0.2 to 3 minutes, preferably 0.5 to 1.5 minutes, to the pellets obtained and is subsequently dried, in order to obtain an isolating layer.

15. Process according to one of the preceding claims, in which the additional shellac layer(s) comprise standard additives, such as plasticizers, colorants, sugar, flavours, antioxidants, and the like.

16. Process according to one of the preceding claims, in which the aqueous shellac is completely or partially replaced as binder by povidone, polyacrylates, cellulose, modified cellulose and mixtures thereof.

17. Pharmaceutical composition in the form of pellets comprising, in this sequence, from the inside outwards, (i) a neutral solid core, (ii) if appropriate a first lower layer of a water-soluble shellac, (iii) an active agent layer comprising a tri- or tetracyclic antidepressant, a water-soluble shellac and, if appropriate, a sugar, and (iv) optionally one or more additional layers, the first of these additional layers, which is in contact with the active agent layer, comprising a water-soluble shellac.

18. Pharmaceutical composition according to Claim 17, in which each pellet comprises: (i) a core, (ii) an active agent layer comprising amitriptyline, amitriptylinoxide, amitriptyline-HCl or amitriptylinoxide-HCl as active agent, water-soluble shellac and sugar, and (iii) at least one additional layer.

19. Pharmaceutical composition according to Claim 17 or 18, as delayed release formulation.

20. Pharmaceutical composition according to Claim 17, 18 or 19, in which each pellet comprises, on 406 to 500 mg/g of neutral pellet core: 20 to 400 mg/g of antidepressant, 5 to 35 mg/g of water-soluble shellac, if appropriate 50 to 200 mg/g of talc and, if appropriate, 0.5 to 2.5 mg/g of sugar.

## Revendications

1. Procédé pour la préparation de pilules, contenant un antidépresseur tri- ou tétracyclique, comprenant les étapes consistant à :
(a) mettre à disposition des pilules neutres ;
(b) répandre l'antidépresseur tri- ou tétracyclique et pulvériser en même temps une solution aqueuse, non alcoolique de gomme laque sans air amené pour obtenir une couche de substance active et éventuellement sécher la couche de substance active, et
(c) déposer éventuellement une ou plusieurs autres couches, dans lesquelles la première de ces couches (= couche d'isolement), qui reste en contact avec la couche de substance active, est obtenue par pulvérisation d'une solution aqueuse, non alcoolique de gomme laque.

2. Procédé selon la revendication 1, dans lequel on répand l'antidépresseur à l'étape (b) pendant toute la pulvérisation de la solution aqueuse, non alcoolique de gomme laque.

3. Procédé selon la revendication 1, dans lequel on pulvérise la solution aqueuse, non alcoolique de gomme laque à l'étape (b) sur les pilules neutres en mouvement dans la turbine d'enrobage.

4. Procédé selon l'une des revendications précédentes, dans lequel on effectue l'étape (b) plusieurs fois jusqu'à l'obtention de la teneur souhaitée en antidépresseur par pilule.

5. Procédé selon l'une des revendications précédentes, dans lequel avant la pulvérisation de la couche de substance active à l'étape (b) on dépose une solution aqueuse, non alcoolique de gomme laque sur les pilules neutres pour former une sous-couche et on la sèche éventuellement.

6. Procédé selon l'une des revendications précédentes, dans lequel on dépose une autre couche à l'étape (c) par pulvérisation d'une solution aqueuse, non alcoolique de gomme laque sans substance active.

7. Procédé selon l'une des revendications 1 à 5, dans lequel on ajuste à l'étape (c) une caractéristique de libération retardée par pulvérisation d'une ou de plusieurs autres couches.

8. Procédé selon l'une des revendications précédentes, dans lequel l'antidépresseur tricyclique est choisi dans le groupe, constitué des dérivés iminodibenzyle, des dérivés iminostilbène, des dérivés dibenzocycloheptadiène, des dérivés dibenzocycloheptatriène, des dérivés dibenzodiazépine, des dérivés dibenzoxépine, des dérivés dihydroanthracène et de leurs sels d'addition avec un acide pharmaceutiquement acceptables.

9. Procédé selon la revendication 8, dans lequel l'antidépresseur est choisi dans le groupe, constitué de : imipramine, désipramine, trimipramine, lofépramine, clomipramine, opipramol, amitriptyline, amitripyline oxyde, nortriptyline, noxiptiline, protriptyline, dibenzépine, doxépine, mélitracen, leurs sels d'addition avec un acide halogénohydrique, en particulier HCI, et leurs mélanges.

10. Procédé selon la revendication 9, dans lequel l'antidépresseur est l'amitriptyline, l'amitriptyline oxyde, l'amitriptyline-HCl ou l'amitriptyline oxyde-HCl.

11. Procédé selon l'une des revendications précédentes, dans lequel la solution de gomme laque utilisée à l'étape (b) contient une gomme laque hydrosoluble.

12. Procédé selon l'une des revendications précédentes, dans lequel la solution de gomme laque contient additionnellement un sucre, de préférence du saccharose, du talc et/ou un anti-oxydant.

13. Procédé selon l'une des revendications précédentes, dans lequel on dépose d'abord une solution aqueuse, non alcoolique de gomme laque avec une vitesse de pulvérisation de 2,5 à 20, de préférence de 5 à 10 g/min sur 1000-10⁴ g de pilules neutres, et on répand après un temps de pulvérisation de 0,2 à 3, de préférence 0,5 à 1,5 minutes, l'antidépresseur en une quantité de 1 à 20, de préférence de 5 à 15 g/min.

14. Procédé selon la revendication 13, dans lequel on dépose ensuite sur les pilules obtenues une solution aqueuse, non alcoolique de gomme laque à une vitesse de pulvérisation de 1 à 20, de préférence 3 à 10 g/min pendant 0,2 à 3, de préférence 0,5 à 1,5 minutes et on sèche ensuite pour obtenir une couche d'isolement.

15. Procédé selon l'une des revendications précédentes, dans lequel la ou les autres couches de gomme laque contiennent des additifs usuels comme des assouplissants, des colorants, des sucres, des aromatisants, des anti-oxydants etc.

16. Procédé selon l'une des revendications précédentes, dans lequel on remplace totalement ou partiellement la gomme laque aqueuse comme liant par de la polyvinylpyrrolidone, du polyacrylate, de la cellulose, de la cellulose modifiée et leurs mélanges.

17. Préparation pharmaceutique en forme de pilules, contenant dans cet ordre de l'intérieur vers l'extérieur (i) un noyau solide, neutre, (ii) éventuellement une première sous-couche d'une gomme laque hydrosoluble, (iii) une couche de substance active, comprenant un antidépresseur tri- ou tétracyclique, une gomme laque hydrosoluble et éventuellement un sucre, et (iv) éventuellement une ou plusieurs autres couches, dans lesquelles la première de ces autres couches, qui reste en contact avec la couche de substance active, contient une gomme laque hydrosoluble.

18. Préparation pharmaceutique selon la revendication 17, dans laquelle chaque pilule contient : (i) un noyau, (ii) une couche de substance active, contenant de l'amitriptyline, l'amitripyline oxyde, l'amitriptyline-HCl ou l'amitriptyline oxyde-HCl comme substance active, une gomme laque hydrosoluble et du sucre, et (iii) au moins une autre couche.

19. Composition pharmaceutique selon la revendication 17 ou 18 comme formulation à libération retardée.

20. Composition pharmaceutique selon la revendication 17, 18 ou 19, dans laquelle chaque pilule contient pour 406 à 500 mg/g de noyau de pilule neutre : 20 à 400 mg/g d'antidépresseur, 5 à 35 mg/g de gomme laque hydrosoluble, éventuellement 50 à 200 mg/g de talc et éventuellement 0,5 à 2,5 mg/g de sucre.
